Europäisches Patentamt

European Patent Office

Office européen des brevets

(19)

(11) Veröffentlichungsnummer: **0 301 346 B1**

(12) **EUROPÄISCHE PATENTSCHRIFT**

(45) Veröffentlichungstag der Patentschrift: 27.11.91

(51) Int. Cl.⁵: **C07C** 21/18, C07C 17/20

(21) Anmeldenummer: 88111502.6

(22) Anmeldetag: 18.07.88

(54) **Herstellung von Polyfluorbutenen.**

(30) Priorität: 30.07.87 DE 3725213

(43) Veröffentlichungstag der Anmeldung:
01.02.89 Patentblatt 89/05

(45) Bekanntmachung des Hinweises auf die
Patenterteilung:
27.11.91 Patentblatt 91/48

(84) Benannte Vertragsstaaten:
BE CH DE FR GB IT LI NL

(56) Entgegenhaltungen:
DE-A- 2 701 310
US-A- 3 149 170
US-A- 3 287 425

JOURNAL OF THE AMERICAN CHEMICAL SO-
CIETY, Band 71, 1949, S. 298-300; A. L. HENNE
et al: "Perfluoro-2-butyne and its Hydrogenation Products"

(73) Patentinhaber: BAYER AG

W-5090 Leverkusen 1 Bayerwerk(DE)

(72) Erfinder: Bielefeldt, Dietmar, Dr.
Beuthener Strasse 13
W-4030 Ratingen 6(DE)
Erfinder: Marhold, Albrecht, Dr.
Carl-Duisberg-Strasse 329
W-5090 Leverkusen 1(DE)

**Beschreibung**

Die vorliegende Erfindung betrifft ein Verfahren zur gleichzeitigen Herstellung von 2-Chlor-1,1,1,4,4,4-hexafluorbuten-2 und 1,1,1,2,4,4,4-Heptafluorbuten-2 aus Hexachlorbutadien.

1,1,1,2,4,4,4-Heptafluorbuten-2, im folgenden auch als Heptafluorbuten bezeichnet, ist ein bekanntes Zwischenprodukt zur Herstellung von Chlorfluorbutan, einem stabilen Wärmeübertragungsmittel, sowie von Trifluoressigsäure und Pentafluorpropionsäure (siehe US-PS 3 287 425). Es ist bekannt Heptafluorbuten herzustellen, indem man Hexachlorbutadien mit Alkalifluorid in einem Lösungsmittel, z.B. Dimethylsulfoxid, umsetzt (siehe US-PS 3 287 425). Nachteilig bei diesem Verfahren ist die Verwendung eines Lösungsmittels und das Entstehen von Alkalichloriden, die aufwendig abgetrennt und entsorgt werden müssen.

2-Chlor-1,1,1,4,4,4-hexafluorbuten-2, im folgenden auch als Chlorhexafluorbuten bezeichnet, ist ein bekanntes Zwischenprodukt zur Herstellung von Natriumtrifluoracetat. Es ist bekannt, daß man es herstellen kann, indem man Hexachlorbutadien mit Fluorwasserstoff und elementarem Chlor unter Zugabe von Antimonpentachlorid umsetzt. Die Ausbeuten sind mit 5 bis 10 % sehr gering, da als Hauptprodukt 2,3-Dichlor-1,1,1,4,4,4-hexafluorbuten-2 erhalten wird (siehe US-PS 2 544 857 und C.A. 46, 7987 i bis 7988 a). Man kann unreines Chlorhexafluorbuten auch als Nebenprodukt erhalten, wenn man aus 2,3-Dichlor-1,1,1,4,4,4-hexafluorbuten-2 in einer 10-tägigen Umsetzung durch Reduktion mit Zink Perfluorbutin-2 herstellt (siehe J. A. C. S. 71, 298 (1949)). Für eine technische Herstellung von Chlorhexafluorbuten sind diese Verfahren nicht geeignet. Nachteilig sind insbesondere der Einsatz von elementarem Chlor oder Zink und die geringen Ausbeuten, in denen ein noch verunreinigtes Produkt anfällt.

Es wurde nun ein Verfahren zur gleichzeitigen Herstellung von 1,1,1,2,4,4,4-Heptafluorbuten-2 und 2-Chlor-1,1,1,4,4,4-hexafluorbuten-2gefunden, das dadurch gekennzeichnet ist, daß man Hexachlorbutadien mit Fluorwasserstoff unter Zugabe Katalytischer Mengen eines Titanhalogenids, Antimontrihalogenids und/oder Antimonpentahalogenids umsetzt.

Das zur Durchführung des erfindungsgemäßen Verfahrens als Ausgangsprodukt benötigte Hexachlorbutadien ist im Handel erhältlich, beispielsweise von der Firma Aldrich Chemie, Steinheim, und kann in der handelsüblichen Reinheit eingesetzt werden. Das zweite benötigte Ausgangsprodukt, der Fluorwasserstoff, wird vorzugsweise in wasserfreier Form eingesetzt und ist als solcher ebenfalls im Handel erhältlich.

Der Fluorwasserstoff kann beispielsweise in Mengen von 5 bis 100 Mol, bezogen auf 1 Mol Hexachlorbutadien verwendet werden. Bevorzugt beträgt diese Menge 10 bis 50 Mol pro Mol Hexachlorbutadien.

In das erfindungsgemäße Verfahren wird Titantetrahalogenid, Antimontrihalogenide und/oder Antimonpentahalogenid in katalytischer Menge zugegeben. Diese Menge kann beispielsweise von 0,1 bis 30 Mol-%, bezogen auf Hexachlorbutadien betragen. Bevorzugt ist diese Menge von 1 bis 25 Mol-%.

Als Halogenide kommen dabei beispielsweise Fluoride und Chloride in Frage, insbesondere Titantetrachlorid, Antimontrifluorid, Antimonpentafluorid, Antimonpentachlorid und gemischte Antimonpentahalogenide der summarischen Formel $SbCl_nF_{5-n}$ mit $n = 0,1$ bis $4,9$. Besonders bevorzugt ist der Zusatz von Antimonpentachlorid. Man kann auch Gemische verschiedener Halogenide einsetzen.

Das erfindungsgemäße Verfahren kann bei verschiedenen Temperaturen durchgeführt werden, beispielsweise bei solchen im Bereich von -20 bis +200°C. Im allgemeinen ist es vorteilhaft bei tiefen Temperaturen zu beginnen, beispielsweise bei -10 bis +18°C und die Reaktion bei höheren Temperaturen, beispielsweise bei 40 bis 160°C zu Ende zu führen. Da Fluorwasserstoff bei Normaldruck bei ungefähr 20°C siedet, ist es beim Arbeiten bei Temperaturen oberhalb etwa 18°C erforderlich, entweder in geschlossenen Gefäßen unter dem sich jeweils ergebenden Eigendruck zu arbeiten und/oder das Verdampfen des Fluorwasserstoffs durch Aufdrücken eines Fremdgases, beispielsweise Stickstoff, zu verhindern. Der entstehende Chlorwasserstoff kann gegebenenfalls über ein Druckhalteventil entspannt werden.

Es ist im allgemeinen vorteilhaft nach Beendigung der Reaktion noch einige Zeit bei der zuletzt angewendeten Temperatur nachzurühren, beispielsweise 1 bis 5 Stunden.

Die Aufarbeitung des Reaktionsgemisches kann so erfolgen, daß man zunächst noch vorhandenen Fluorwasserstoff abtrennt, z.B. durch Phasentrennung oder durch Destillation, und den Rückstand fraktioniert destilliert oder den vom Fluorwasserstoff befreiten Rückstand auf Eis gibt, die sich bildende organische Phase abtrennt und diese fraktioniert destilliert. Der Katalysator kann gegebenenfalls, z.B. durch Extraktion mit einer Weinsäurelösung aus den nach der Entfernung des Fluorwasserstoffs vorliegenden Rückstand, abgetrennt werden.

Auf diese Weise kann man Heptafluorbuten und Chlorhexafluorbuten in verschiedenen Verhältnisses zueinander erhalten und beide Produkte in sehr reiner Form isolieren. Im allgemeinen enthält das rohe Reaktionsgemisch mehr Chlorhexafluorbuten als Heptafluorbuten.

Es ist ausgesprochen überaschend, daß Heptafluorbuten mit Fluorwasserstoff und ohne Lösungsmittel und Chlorhexafluorbuten ohne die Verwendung von elementarem Chlor auf die erfindungsgemäße Weise so vorteilhaft erhalten werden können.

Beispiele

Beispiel 1

Zu 4,5 l Fluorwasserstoff, die mit 74 ml Antimonpentachlorid versetzt worden waren, wurden bei 0°C 1400 ml Hexachlorbutadien hinzugefügt. Nach Beendigung der Chlorwasserstoffentwicklung wurden 25 bar Stickstoff aufgedrückt und die Temperatur auf 120°C gesteigert. Bei dieser Temperatur wurde 4 Stunden lang gerührt und der entstehende Chlorwasserstoff über ein Druckhalteventil entspannt. Danach wurde nicht-umgesetzter Fluorwasserstoff abdestilliert, der Rückstand auf Eis gegeben und fraktioniert destilliert. Es wurden erhalten:
790 g (65 % der Theorie) 2-Chlor-1,1,1,4,4,4-hexafluorbuten-2 mit einem Siedepunkt von 36°C bei 1 bar und 20 g (2 % der Theorie 1,1,1,2,4,4,4-Heptafluorbuten-2 mit einem Siedepunkt von 9°C bei 1 bar.

Außerdem wurden 620 g nicht-umgesetztes Hexachlorbutadien zurückgewonnen.

Die Charakterisierung der isolierten Substanzen erfolgte durch kernmagnetische Resonanzspektren und Massenspektren.

Beispiel 2

Zu 3 l Fluorwasserstoff und 50 ml Antimonpentachlorid wurden bei -4°C 720 ml Hexachlorbutadien hinzugefügt. Das Reaktionsgemisch wurde langsam auf 17°C erwärmt. Nach dem Ende der Chlorwasserstoffentwicklung wurden 30 bar Stickstoff aufgedrückt, das Gemisch 3 Stunden bei 140°C gerührt und der entstehende Chlorwasserstoff über ein Druckhalteventil entspannt. Danach wurde das Produktgemisch abgekühlt und durch Destillation gereinigt. Es wurden erhalten: 198 g (= 23,5 % der Theorie) 1,1,1,2,4,4,4-Heptafluorbuten-2 und 521 g (= 56,6 % der Theorie) 2-Chlor-1,1,1,4,4,4-hexafluorbuten-2.

Die Siedepunkte und die Charakterisierung waren wie im Beispiel 1 angegeben. Außerdem wurden 20 g nichtumgesetztes Hexachlorbutadien zurückgewonnen.

Beispiel 3

Zu 3 l Fluorwasserstoff, die mit 150 ml Antimonpentachlorid und 50 g Antimontrifluorid versetzt worden waren, wurden bei -2°C 720 ml Hexachlorbutadien hinzugefügt. Dieses Gemisch wurde 6,5 Stunden bei 20° gehalten. Danach wurden 25 bar Stickstoff aufgedrückt und die Temperatur auf 138°C gesteigert. Bei dieser Temperatur wurde 4,5 Stunden gerührt und der entstehende Chlorwasserstoff über ein Druckhalteventil entspannt. Nach dem Abtrennen von nicht umgesetztem Fluorwasserstoff wurde der Rückstand fraktioniert destilliert. Es wurden erhalten: 340 g (= 42 % der Theorie) 1,1,1,2,4,4,4-Heptafluorbuten-2 und 485 g (= 54 % der Theorie) 2-Chlor-1,1,1,4,4,4-Hexafluorbuten-2.

Die Siedepunkte und die Charakterisierung waren wie in Beispiel 1 angegeben. Außerdem wurden 40 g nicht umgesetztes Hexachlorbutadien zurückgewonnen.

Patentansprüche

1. Verfahren zur gleichzeitigen Herstellung von 1,1,1,2,4,4,4-Heptafluorbuten-2 und 2-Chlor-1,1,1,4,4,4-Hexafluorbuten-2,dadurch gekennzeichnet, daß man Hexachlorbutadien mit Fluorwasserstoff unter Zugabe katalytischer Mengen eines Titanhalogenids, Antimontrihalogenids und/oder Antimonpentahalogenids umsetzt.

2. Verfahren nach Anspruch 1, dadurch gekennzeichnet, daß man 5 bis 100 Mol Fluorwasserstoff pro Mol Hexachlorbutadien verwendet.

3. Verfahren nach Ansprüchen 1 und 2, dadurch gekennzeichnet, daß man 0,1 bis 30 Mol-% eines Titanhalogenids, Antimontrihalogenids und/oder Antimonpentahalogenids, bezogen auf Hexachlorbutadien, einsetzt.

4. Verfahren nach Ansprüchen 1 bis 3, dadurch gekennzeichnet, daß man es bei Temperaturen im Bereich -20 bis +200°C durchführt.

5. Verfahren nach Ansprüchen 1 bis 4, dadurch gekennzeichnet, daß man bei Temperaturen oberhalb 18°C in geschlossenen Gefäßen und/oder unter Aufdrücken von Fremdgas arbeitet.

6. Verfahren nach Ansprüchen 1 bis 5, dadurch gekennzeichnet, daß man aus dem Reaktionsgemisch zunächst noch vorhandenen Fluorwasserstoff abtrennt und danach den Rückstand fraktioniert destilliert.

Claims

1. Process for the simultaneous preparation of 1,1,1,2,4,4,4-heptafluoro-2-butene and 2-chloro-1,1,1,4,4,4-hexafluoro-2-butene, characterised in that hexachlorobutadiene is reacted with hydrogen fluoride with the addition of catalytic amounts of a titanium halide, antimony trihalide and/or antimony pentahalide.

2. Process according to Claim 1, characterised in that 5 to 100 mol of hydrogen fluoride are used per mole of hexachlorobutadiene.

3. Process according to Claims 1 and 2, characterised in that 0.1 to 30 mol % of a titanium halide, antimony trihalide and/or antimony pentahalide, relative to hexachlorobutadiene, are used.

4. Process according to Claims 1 to 3, characterised in that it is carried out at temperatures in the range from -20 to +200°C.

5. Process according to Claims 1 to 4, characterised in that it is carried out at temperatures above 18°C in closed vessels and/or by pressurising with another gas.

6. Process according to Claims 1 to 5, characterised in that any hydrogen fluoride still present is first removed from the reaction mixture and the residue is then subjected to fractional distillation.

**Revendications**

1. Procédé pour la préparation simultanée du 1,1,1,2,4,4,4-heptafluorobutène-2 et du 2-chloro-1,1,1,4,4,4-hexafluorobutène-2, **caractérisé en ce qu'**on fait réagir de l'hexachlorobutadiène avec du fluorure d'hydrogène avec addition de quantités catalytiques d'un halogénure de titane, d'un trihalogénure d'antimoine et/ou d'un pentahalogénure d'antimoine.

2. Procédé selon la revendication 1, **caractérisé en ce qu'**on utilise de 5 à 100 moles de fluorure d'hydrogène par mole d'hexachlorobutadiène.

3. Procédé selon les revendications 1 et 2, **caractérisé en ce qu'**on met en oeuvre de 0,1 à 30 moles % d'un halogénure de titane, d'un trihalogénure d'antimoine et/au d'un pentahalogénure d'antimoine, rapporté sur l'hexachlorobutadiène.

4. Procédé selon les revendications 1 à 3, **caractérisé en ce qu'**on l'effectue à des températures dans le domaine de -20 à +200°C.

5. Procédé selon les revendications 1 à 4, **carac-**

térisé en ce qu'on travaille, à des températures supérieures à 18°C, dans des récipients fermés et/ou sous pression d'un gaz étranger.

6. Procédé selon les revendications 1 à 5, **caractérisé en ce qu'**on sépare d'abord du mélange réactionnel le fluorure d'hydrogène encore présent et ensuite on soumet le résidu à une distillation fractionnée.